# EUROPÄISCHE PATENTSCHRIFT

(11) **EP 0 439 768 B1**
(45) Veröffentlichungstag und Bekanntmachung des Hinweises auf die Patenterteilung: **27.11.1996**
(21) Anmeldenummer: 90124317.0
(22) Anmeldetag: 15.12.1990
(51) Int. Cl.: B65H 63/00, G01N 33/36

(54) **Verfahren zur qualitativen Klassierung von elektronisch gereinigtem Garn**
Method for qualitative classification of electronically cleaned yarn
Méthode pour la classification qualitative des fils nettoyés électroniquement

(30) Priorität: 26.01.1990 CH 259/90
(43) Veröffentlichungstag der Anmeldung: 07.08.1991
(73) Patentinhaber: ZELLWEGER LUWA AG, CH-8610 Uster (CH)
(72) Erfinder: Aemmer, Peter F., Dr., CH-8907 Wettswil (CH)

(56) Entgegenhaltungen:
- DE-A- 3 712 654
- DE-A- 4 003 810
- US-A- 3 731 069
- US-A- 4 430 720
- USTER NEWS BULLETIN, Heft 29, August 1981

## Beschreibung

Die vorliegende Erfindung betrifft ein Verfahren zur Klassierung und Reinigung von Garn im Hinblick auf Garnfehler, bei welchem das Garn einen Messkopf durchläuft, der das Garn abtastet und dabei Signale abgibt, die von Qualitätsmerkmalen des Garns und allfälliger Garnfehler abhängen.

Die elektronische Garnreinigung ist seit langem bekannt und beispielsweise in der Publikation USTER News Bulletin (USTER - eingetragenes Warenzeichen der Zellweger Uster AG) Nr. 29 vom August 1981 ausführlich beschrieben. Dieser Publikation ist unter anderem zu entnehmen, dass bei der elektronischen Garnreinigung das richtige Verhältnis gefunden werden muss zwischen Fehlern, die man im Garn belassen kann, und solchen, die durch einen Knoten ersetzt werden müssen. Das bedeutet, dass auch ein gereinigtes Garn noch Garnfehler aufweist, und zwar solche, die bei der Reinigereinstellung als tolerierbar eingestuft wurden. Somit besteht in Hinblick auf diese nicht ausgereinigten Garnfehler ein gewisser Qualitätsunterschied zwischen verschiedenen Spulen mit gereinigtem Garn. Selbstverständlich besteht ein solcher Qualitätsunterschied auch bezüglich anderer Parameter, wie zum Beispiel der Anzahl Spleisse oder Knoten, der Anzahl Nissen, oder des Variationskoeffizienten der Gleichmässigkeit (CV%). Ausserdem besteht auch ein Unterschied bezüglich der Garnfehlerklassen, wie sie beispielsweise beim USTER CLASSIMAT verwendet werden. Der USTER CLASSIMAT ist ebenfalls in dem schon erwähnten USTER News Bulletin Nr. 29 beschrieben.

Aus der US-A-4,430,720 ist ein Verfahren zum Reinigen von Garnen bekannt, bei dem eine Bewertung der Garnfehler nur zum Zwecke der Reinigung dieser Fehler vorgenommen wird. Dabei sind die für die Reinigung festgelegten Grenzen für die Garnfehler so gelegt, dass sie etwa den Garnfehlerklassen entsprechen, wie sie aus dem USTER CLASSIMAT bekannt sind.

Aus der US-A-3,731,009 ist ferner ein Verfahren zum Erfassen der Garnqualität bekannt, bei dem Qualitätsangaben nur zum Ueberwachen der Qualität und zum Beseitigen von Fehlern im Garn herangezogen werden.

Der beschriebene Umstand, dass zwei mit dem gleichen Reiniger und mit der gleichen Einstellung gereinigte Spulen in aller Regel nicht die gleiche Qualität aufweisen werden, führt zur Ueberlegung, dass es für einen Garnhersteller wünschenswert sein könnte, Spulen mit gereinigtem Garn nach Qualitätskriterien unterscheiden zu können. Denn das würde die interessante Möglichkeit eröffnen, Markenware verschiedener, beispielsweise normaler und gehobener, Qualität anbieten zu können, wobei die gehobene Markenware zu besseren Preisen abgesetzt werden könnte.

Die an sich naheliegende qualitative Unterscheidung von nach gemeinsamen Parametern gereinigten Garnspulen anhand der Resultate einer on-line Klassierung in eine Vielzahl von Garnfehlerklassen oder anhand der CLASSIMAT-Klassen oder eines ähnlichen Schemas ist aber relativ aufwendig und noch dazu textiltechnisch nicht sehr sinnvoll. Denn da die groben Fehler ohnehin ausgereinigt werden, bleiben nur wenige aussagekräftige Klassen übrig. Wenn aber nur wenige Klassen relevant sind, dann resultiert ein Missverhältnis zwischen dem technischen Aufwand für eine vollständige Klassierung und deren potentiellem Ergebnis. Dazu kommt noch, dass bei nur wenigen Klassen die Differenzierungsmöglichkeit schlecht ist, da das auf die wenigen Klassen bezogene "Qualitätsraster" zu grob wird.

Durch die Erfindung soll nun ein Verfahren angegeben werden, welches eine aussagekräftige und wiederholbare qualitative Klassierung von elektronisch gereinigtem Garn ermöglicht, und zwar mit möglichst geringem technischem und kostenmässigem Aufwand.

Diese Aufgabe wird erfindungsgemäss dadurch gelöst, dass die Querschnittssignale zusätzlich mit den genannten Reinigungsgrenzen zugeordneten Klassierungsgrenzen verglichen werden, welche jeweils enger sind als die entsprechenden Reinigungsgrenzen, und dass jedes Ueberschreiten einer Klassierungsgrenze für das jeweilige Qualitätsmerkmal eine den betreffenden Garnabschnitt unbeeinflusst lassende Registrierung auslöst.

Das erfindungsgemässe Verfahren stellt also eine Art von virtueller Garnreinigung dar, welche gegenüber der konventionellen, echten Garnreinigung zwei Unterschiede aufweist: Einerseits sind die Grenzwerte der virtuellen Garnreinigung enger und schärfer als diejenigen der echten, und andererseits löst eine Ueberschreitung dieser als Klassierungsgrenze bezeichneten Grenzwerte keinen Reinigerschnitt aus, sondern bloss eine Registrierung, also beispielsweise einen Zählschritt eines Zählers.

Der Garnhersteller erhält mit dem erfindungsgemässen Verfahren die Möglichkeit, mit nur sehr geringem Mehraufwand eine Aussage über die Qualität des hergestellten Garns machen zu können, indem die Qualitätskriterien durch die sozusagen nicht ausgereinigten "Garnfehler" gebildet werden. Wie die echte Garnreinigung erfolgt auch die virtuelle in mehreren Kanälen, entsprechend verschiedenen Qualitätsmerkmalen wie lange und kurze Dick- und Dünnstellen, so dass beide Verfahren voll miteinander vergleichbar sind. Ein für den Garnhersteller weiterer wesentlicher Vorteil besteht darin, dass sich dieser auf einem ihm von der bekannten Garnreinigung her vertrauten Gebiet bewegt. Das heisst, dass ihm die Einstellungsparameter und ihr Zusammenhang mit Garnfehlern und deren Häufigkeit vertraut sind und er daher für das neue Verfahren nichts dazulernen oder umdenken muss.

In der US-A-4 430 720 ist ein Verfahren zur Reinigung von Garnen und zur Bewertung von Garnfehlern beschrieben. Diesem Patent lässt sich entnehmen, dass der vom zu reinigenden Garn durchlaufene Messkopf ein Spannungssignal abgibt, dessen Wert jederzeit ein Mass für den Querschnitt oder den Durchmesser des gerade abgetasteten Signals ist. Dieses Signal wird ständig daraufhin untersucht, ob es innerhalb eines vorgegebenen Toleranzbereichs liegt oder diesen überschreitet, und es wird ausserdem die Länge jeder derartigen Ueberschreitung festgestellt.

Es ist in diesem Patent ferner gezeigt, dass eine Vorrichtung zur Durchführung des beschriebenen Verfahrens aus einer Anzahl von Stufen besteht, von denen jede einer eine Anzahl Spulstellen umfassenden Sektion einer Spulmaschine zugeordnet und an ihrem Eingang vom Garnabtastsignal eines Messkopfes gespeist ist. Jede Stufe besitzt einen Hauptausgang, an welchem ein Signal erscheint, das zur Steuerung einer Garntrennvorrichtung verwendet werden kann, Nebeneingänge für externe Befehle und Signale und Nebenausgänge für statistische Daten über die Produktion der entsprechenden Spulstelle oder davon abgeleitete Steuerungs- oder Warnfunktionen. Ausserdem haben alle Stufen Zugriff zu einem zentralen Speicherbereich, wo die diversen zur Signalanalyse und zur Auslösung von Steuerungs- und Warnfunktionen notwendigen Parameter, wie insbesondere die Toleranzgrenzen für Quer- und Längsdimension, gespeichert sind.

Beim erfindungsgemässen Verfahren wird nun nichts anderes gemacht, als dass jeder Toleranzbereich oder jedenfalls gewissen Toleranzbereichen, deren Toleranzgrenzen die sogenannten Reinigungsgrenzen bilden, ein zweiter, engerer Toleranzbereich mit schärferen Toleranzgrenzen zugeordnet wird, dessen Parameter im zentralen Speicherbereich gespeichert werden. Während dann, wenn das Garnabtastsignal den weiteren Toleranzbereich mit den Reinigungsgrenzen verlässt, gegebenenfalls ein Schnittvorgang ausgelöst wird, bewirkt ein Ueberschreiten des engeren Toleranzbereichs durch das Garnabtastsignal lediglich eine entsprechende Fehlerregistrierung.

Die genannten Fehlerregistrierungen werden für die betreffenden Qualitätsmerkmale getrennt gezählt, so dass man für jeden Kanal eine Summe erhält. Die so erhaltenen Einzelsummen werden entsprechend gewichtet und es wird daraus eine Gesamtsumme gebildet, die dann das Kriterium für die qualitative Klassierung bildet.

Dieses nachfolgend als "virtuelle" Reinigung bezeichnete Verfahren erfolgt so wie die echte Garnreinigung mit den bekannten Kanälen oder Fehlerarten kurze und lange Dick- und Dünnstellen, und es ist selbstverständlich dem Garnhersteller freigestellt, wie er die Grenzen einstellt. Es hat sich gezeigt, dass eine auf kurze Dick- und Dünnstellen empfindlich eingestellte virtuelle Reinigung für gewisse Garnhersteller, welche Garn an Hersteller von Prestigemarken liefern, sehr interessant sein dürfte, weil Gewebe und Gewirke mit vergleichsweise vielen kurzen Dick- und Dünnstellen nach der Einfärbung ein deutlich unruhigeres Warenbild aufweisen.

In mikroprozessorbasierten Garnreinigern ist die geschilderte virtuelle Reinigung besonders einfach zu realisieren, da der erforderliche Mehraufwand einzig durch die notwendigen Algorithmen gegeben ist. Da diese aber mit denjenigen der regulären Garnreinigung praktisch identisch sind und sich ausserdem ausführungsmässig gut mit diesen kombinieren lassen, ist der Mehraufwand, insbesondere gemessen am erzielbaren Ergebnis, ausserordentlich bescheiden.

Für eine virtuelle Reinigung auf der Basis von kurzen Dick- und Dünnstellen sind besonders Anlagen mit Messköpfen, welche das kapazitive Messprinzip anwenden, sehr gut geeignet. Dagegen sind optische Messsysteme dafür weniger gut bis gar nicht geeignet, besonders wenn es sich bei diesen um eine reine Durchmessermessung handelt. Denn bei einer solchen wird der bekannte und unvermeidliche Formeffekt das Messergebnis bei kurzen Bezugslängen als überlagerte Störung sehr stark beeinträchtigen.

Auch nach der schon zitierten USTER CLASSIMAT Methode oder einem ähnlichen Verfahren, bei dem die möglichen Fehler nach Länge und Querschnitt in 23 Klassen, davon 16 Klassen für kurze Dickstellen und keine Klasse für kurze Dünnstellen, klassiert werden, ist eine differenzierte Qualitätseinteilung nach kurzen Dick- und Dünnstellen nicht möglich.

Dagegen bietet die virtuelle Reinigung die Möglichkeit, elektronisch gereinigtes Garn nach Qualitätskriterien zu differenzieren und diese Differenzierung in wertmehrende Marktvorteile umzusetzen. Zu diesem Zweck muss maschinenseitig dafür gesorgt werden, dass die betreffenden Spulen gemäss dem Resultat der virtuellen Reinigung entsprechend markier- und/oder identifizierbar sind. Man kann dazu die Spulen mit einer Markierung in Form eines Aufdrucks oder einer Etikette versehen und/oder man kann an der Spulmaschine eine Weiche vorsehen und die qualitativ unterschiedlichen Spulen zwangsläufig auf entsprechende Geleise transportieren. Auf diese Weise liegen dann die Spulen bereits nach Qualität sortiert vor, und man wird die bessere Qualität zu entsprechend besseren Preisen absetzen können.

## Patentansprüche

1. Verfahren zur Klassierung und Reinigung von Garn im Hinblick auf Garnfehler, bei welchem das Garn einen Messkopf durchläuft, der das Garn abtastet und dabei Signale abgibt, die von Qualitätsmerkmalen des Garns und allfälliger Garnfehler abhängen, dadurch gekennzeichnet, dass für mindestens ein Qualitätsmerkmal des Garns Toleranzgrenzen vorgegeben werden, die einen funktionalen Zusammenhang zwischen Parametern des Garns und allfälliger Garnfehler herstellen, dass eine Toleranzgrenze für die Reinigung und mindestens eine weitere Toleranzgrenze für eine Klassierung des Garns bestimmt wird, dass die Signale vom Messkopf gleichzeitig mit den Toleranzgrenzen für Reinigung und Klassierung verglichen werden, dass die Signale, die die Toleranzgrenze für die Reinigung überschreiten einen Garnfehler feststellen, der nachfolgend aus dem Garn herausgeschnitten wird und dass die Signale, die eine Toleranzgrenze für die Klassierung überschreiten, einen Garnfehler feststellen, der nachfolgend registriert aber nicht herausgeschnitten wird.

2. Verfahren nach Anspruch 1, dadurch gekennzeichnet, dass als Parameter die prozentuale Abweichung des Garndurchmessers von einem Mittelwert und die Länge einer solchen Abweichung vorgesehen sind.

3. Verfahren nach Anspruch 1, dadurch gekennzeichnet, dass als Parameter die Anzahl Knoten, Spleisse oder Nissen und die Länge eines Garnabschnittes vorgesehen sind.

4. Verfahren nach einem der Ansprüche 1 bis 3, dadurch gekennzeichnet, dass nach Reinigung und Klassierung das Garn auf Spulen aufgewickelt wird und dass die Spulen mit dem gereinigten Garn anhand des Ergebnisses der Klassierung in verschiedene Qualitätsklassen eingeteilt und entsprechend bezeichnet und/oder sortiert werden.

## Claims

1. Method for the classification and clearing of yarn in respect of yarn faults, in which the yarn passes through a measuring head which scans the yarn and in so doing emits signals which are dependent on quality features of the yarn and miscellaneous yarn faults, characterized in that, for at least one quality feature of the yarn, tolerance limits are stipulated, which provide a functional correlation between parameters of the yarn and miscellaneous yarn faults, in that a tolerance limit is determined for the clearing and at least one further tolerance limit is determined for classification of the yarn, in that the signals from the measuring head are simultaneously compared with the tolerance limits for clearing and classification, in that the signals which exceed the tolerance limit for the clearing detect a yarn fault which is subsequently cut out of the yarn, and in that the signals which exceed a tolerance limit for the classification detect a yarn fault which is subsequently registered, but not cut out.

2. Method according to Claim 1, characterized in that the percentage deviation of the yarn diameter from a mean value and the length of such a deviation are provided as parameters.

3. Method according to Claim 1, characterized in that the number of knots, splices or burls and the length of a yarn section are provided as parameters.

4. Method according to one of Claims 1 to 3, characterized in that, after clearing and classification, the yarn is wound onto bobbins, and in that the bobbins of the cleared yarn are categorized into different quality classes on the basis of the result of the classification and are correspondingly designated and/or sorted.

## Revendications

1. Procédé pour la classification et l'épuration de fils en vue de défauts de fil, où le fil passe à travers une tête de mesure qui balaie le fil et, ce faisant, émet des signaux qui dépendent des caractéristiques de qualité du fil et d'éventuels défauts de fil caractérisé en ce que, pour au moins une caractéristique de qualité du fil, des limites de tolérance sont prédéfinies qui établissent un rapport fonctionnel entre des paramètres du fil et des défauts de fil éventuels, en ce qu'il est défini une limite de tolérance pour l'épuration et au moins une autre limite de tolérance pour une classification du fil, en ce que les signaux de la tête de mesure sont comparés simultanément avec des limites de tolérance pour l'épuration et la classification, en ce que les signaux qui dépassent la limite de tolérance pour l'épuration constatent un défaut de fil qui sera coupé ensuite du fil, et en ce que les signaux qui dépassent une limite de tolérance pour la classification, constatent un défaut de fil qui sera enregistré ensuite mais qui ne sera pas découpé.

2. Procédé selon la revendication 1, caractérisé en ce que sont prévus, comme paramètres, l'écart en pour cent du diamètre du fil d'une valeur moyenne et la longueur d'un tel écart.

3. Procédé selon la revendication 1, caractérisé en ce que sont prévus, comme paramètres, le nombre de noeuds ou d'épissures, et la longueur d'un tronçon de fil.

4. Procédé selon l'une des revendications 1 à 3, caractérisé en ce qu'après l'épuration et la classification, le fil est enroulé sur des bobines et en ce que les bobines avec le fil épuré sont réparties à l'aide du résultat de la classification dans des classes de qualité différentes et sont marquées et/ou triées de façon correspondante.
